(19) Europäisches Patentamt
European Patent Office
Office européen des brevets

(11) Veröffentlichungsnummer: **0 069 198**
**B1**

(12) **EUROPÄISCHE PATENTSCHRIFT**

(45) Veröffentlichungstag der Patentschrift: **15.01.86**

(51) Int. Cl.⁴: **C 07 D 307/935**, A 61 K 31/34

(21) Anmeldenummer: **82103074.9**

(22) Anmeldetag: **09.04.82**

(54) **Verfahren zur Herstellung von 6-substituierten 7-Oxo-PGI-Derivaten.**

(30) Priorität: **14.04.81 HU 96881**

(43) Veröffentlichungstag der Anmeldung: **12.01.83 Patentblatt 83/2**

(45) Bekanntmachung des Hinweises auf die Patenterteilung: **15.01.86 Patentblatt 86/3**

(84) Benannte Vertragsstaaten: **BE DE FR GB IT LU NL**

(56) Entgegenhaltungen: **EP-A-0 031 426**

(73) Patentinhaber: **CHINOIN Gyogyszer és Vegyészeti Termékek Gyára RT., To utca 1-5, H-1045 Budapest IV (HU)**

(72) Erfinder: **Tömösközi, István, Dr., Pozsonyi u. 6/a, H-1045 Budapest (HU)**
Erfinder: **Székely, István, Dr., Krajcár u. 6, H-2120 Dunakeszi (HU)**
Erfinder: **Kánai, Károly, Dipl.- Ing., Markó u. 7, H-1055 Budapest (HU)**
Erfinder: **Györi, Péter, Dr., Dipl.- Ing., Gyakorló u. 32, H-1106 Budapest (HU)**
Erfinder: **Kovács, Gábor, Dr., Dipl.- Ing., Róna park 4, H-1142 Budapest (HU)**

(74) Vertreter: **Lotterhos, Hans Walter, Dr.- Ing., Lichtensteinstrasse 3, D-6000 Frankfurt am Main 1 (DE)**

EP 0 069 198 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein neues, einfaches und wirtschaftliches Verfahren zur Herstellung von 6:substituierten-7-Oxo-PGI-Derivaten der allgemeinen Formel

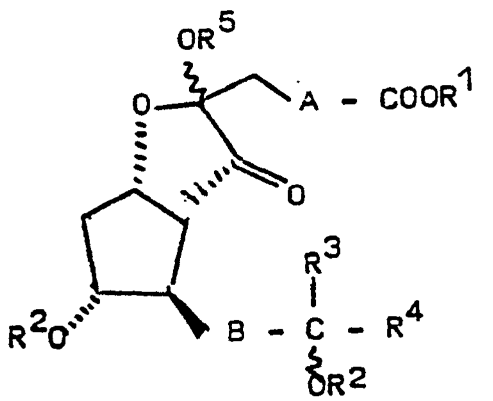

(I) ,

worin
A geradkettiges oder verzweigtes $C_{1-5}$-Alkylen,
B Äthylen, Z- oder E-Vinylen oder Äthinylen,
$R^1$ Wasserstoff, $C_{1-5}$-Alkyl oder ein pharmazeutisch verträgliches Kation,
$R^2$ Wasserstoff, $C_{1-5}$-Alkanoyl oder Aroyl,
$R^3$ Wasserstoff oder Methyl,
$R^4$ geradkettiges oder verzweigtes $C_{1-8}$-Alkyl oder gegebenenfalls monosubstituiertes Aryloxymethyl und
$R^5$ Wasserstoff oder $C_{1-5}$-Alkyl bedeuten

Die Verbindungen der allgemeinen Formel I können racemisch oder optisch aktiv sein. Die Stereokonfiguration der durch das Zeichen "~" bezeichneten ~ $OR^2$- und ~ $OR^5$-Substituenten kann α oder β sein.

Obwohl die Verbindungen der allgemeinen Formel I selbst über eine gewisse biologische Aktivität verfügen, können sie in erster Linie als wichtige synthetische Zwischenprodukte zur Herstellung von 7-Oxo-$PGI_2$-Derivaten der allgemeinen Formel

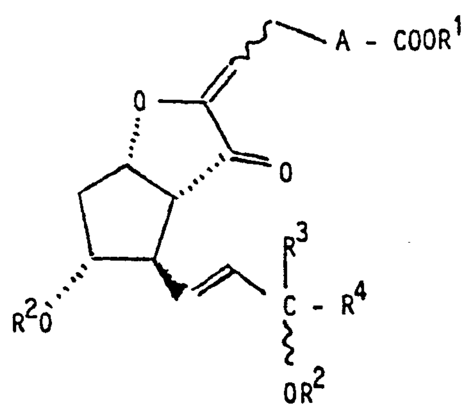

(II) ,

worin
A, B, $R^1$, $R^2$, $R^3$ und $R^4$ die oben angegebene Bedeutung haben, verwendet werden.

Die Herstellung der Verbindungen der allgemeinen Formel II ist in der unter der Nr. 0 031 426 veröffentlichten europäischen Patentanmeldung beschrieben. Diese Verbindungen üben eine starke und pharmazeutisch wertvolle blutaggregationshemmende, disaggregierende, blutdrucksenkende, koronarerweiternde, antianginöse, cytoprotektive und antidiarrhoetische Wirkung aus und aktivieren das Adenylat-Cyclase-Enzymsystem der Blutplättchen.

Die Verbindungen der allgemeinen Formel II können aus den Verbindungen der allgemeinen Formel 1 durch Eliminierung der Elemente der Verbindung der allgemeinen Formel $R^5OH$ hergestellt werden (wenn $R^5$ Wasserstoff ist, wird Wasser, wenn $R^5$ Alkyl bedeutet, wird Alkohol abgespalten). Diese Verfahren sind in der oben angegebenen europäischen Patentanmeldung be-schrieben.

Die Herstellung der Verbindungen der allgemeinen Formel II ist nicht Gegenstand der vorliegenden Erfindung.

Die bisher bekannten Verfahren zur Herstellung der Verbindungen der allgemeinen Formel I sind mit schwerwiegenden Nachteilen verbunden. Diese bekannten Verfahren stellen umständliche, aus vielen Stufen bestehende Synthesen dar.

Es wurde überraschenderweise gefunden, daß die Verbindungen der allgemeinen Formel I einfach und in guten Ausbeuten hergestellt werden können, wenn man eine der leicht zugänglichen, bekannten Verbindungen der allgemeinen Formel

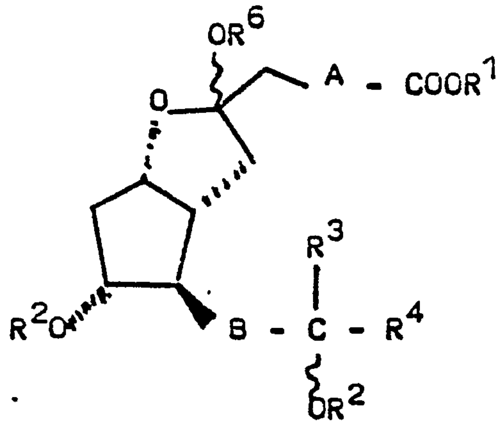

(III),

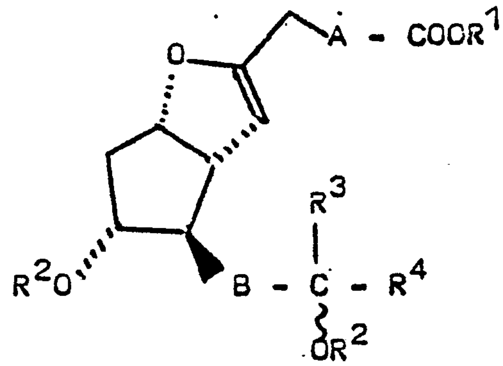

(IV)

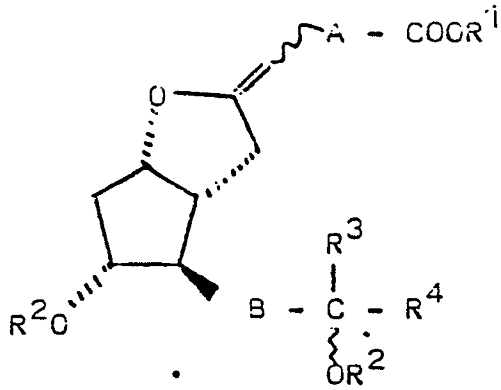

(V),

worin

A, B, $R^1$, $R^2$, $R^3$ und $R^4$ die oben angegebene Bedeutung haben und $R^6$ Wasserstoff oder $C_{1-5}$-Alkyl oder Alkanoyl bedeutet, oder ein Gemisch dieser Verbindungen mit einem milden elektrophilen Oxydationsmittel in einem Gemisch von Wasser und einem organischen Lösungsmittel oxydiert. Bei dieser Oxydation erhält man Verbindungen der allgemeinen Formel I, in denen $R^5$ Wasserstoff bedeutet. In diesen Verbindungen der allgemeinen Formel I kann der Wasserstoff ($R^5$) durch Umsetzung mit einem $C_{1-5}$-Alkanol in Gegenwart von Bortrifluoridätherat durch $C_{1-5}$-Alkyl ersetzt werden.

Die Ausgangsverbindungen der allgemeinen Formeln III, IV und V sind entweder bekannt oder können in analoger Weise wie die bekannten Derivate ähnlicher Struktur hergestellt werden.

Die Ausgangsverbindungen der allgemeinen Formel III können z.B. aus den entsprechenden substituierten $PGF_{2\alpha}$ -Derivaten durch Umsetzung mit Wasser oder mit einem $C_{1-5}$-Alkanol in Gegenwart von 1 - 2 Mol einer Thallium(III)Verbindung - pro 1 Mol $PGF_{2\alpha}$ -Derivat - hergestellt werden. Je nach dem, ob bei dieser Umsetzung Wasser oder Alkanole verwendet werden, werden Verbindungen der allgemeinen Formel III, in denen $R^6$ Wasserstoff oder $C_{1-5}$-Alkyl ist, erhalten. Diese Verbindungen der allgemeinen Formel III, in denen $R^6$ $C_{1-5}$-Alkanoyl ist, können aus den entsprechenden Verbindungen der allgemeinen Formel III, in denen $R^6$ Wasserstoff ist, z.B. durch Umsetzung mit dem Anhydrid oder Säurechlorid der entsprechenden $C_{1-5}$-Alcancarbonsäure in Gegenwart eines tertiären Amins hergestellt werden.

Der einfachste Vertreter der Ausgangsverbindungen der allgemeinen Formel IV ist das $\Delta^6$-$PGI_1$ ($R^1$, $R^2$ und $R^3$ sind Wasserstoff, A ist eine $-(CH_2)_3$-Gruppe, B ist E-Vinylen und $R^4$ n-Pentyl). Die Herstellung dieser Verbindung ist in J.Am.Chem.Soc. 100, 2547 und 7690 (1978) beschrieben. Die anderen Ausgangsverbindungen der allgemeinen Formel IV können in analoger Weise aus den entsprechend substituierten Ausgangsverbindungen hergestellt werden.

Der bekannteste Vertreter der Ausgangsverbindungen der allgemeinen Formel V ist das Prostacyclin. Die Herstellung dieser Verbindung und deren Analoga ist z.B. in J.Am.Chem.Soc. 100, 7690 (1978) und in der DE-OS 2 702 553 beschrieben. Die in diesen Literaturstellen nicht beschriebenen weiteren Derivate können nach bekannten Verfahren aus den entsprechenden substituierten Ausgangsverbindungen hergestellt werden.

Aus der nicht vorveröffentlichten WO 82/01 002 ist bekannt, zur Herstellung von 7-Oxo-prostacyclin-Derivaten, die in 6-Stellung unsubstituiert sind, Prostacyclin-Derivate mit Selendioxyd in Gegenwart von einem organischen Lösungsmittel, vorzugsweise wasserfreiem Dioxan, zu oxydieren.

Nach dem Verfahren der Erfindung werden zur Herstellung von 6-substituierten 7-Oxo-PGI-Derivaten der allgemeinen Formel I Verbindungen der allgemeinen Formeln III, IV oder V einzeln oder im Gemisch mit einem milden elektrophilen Oxydationsmittel in einem Gemisch von Wasser und einem organischen Lösungsmittel oxydiert.

Zur Definition der Bezeichnung "mildes elektrophiles Oxydationsmittel" wird auf die Veröffentlichung R.G.Pearson: J.Chem.Educ. 45, 581 und 683 (1968) verwiesen. Zu diesem Zweck kann vorteilhaft Selendioxyd der Formel

$SeO_2$ (VI)

verwendet werden. Die Anwendung dieses Reagenzes ist in den folgenden Literaturstellen: M.J.Reich: Organoselenium Oxidations, Oxidation in Organic Chemistry, Editor: W.S.Trahanovsky, Academic Press Inc. New York, 1978; und D.L.J.Clive: Modern Organoselenium Chemistry, Tetrahedron Report No. 50, Tetrahedron 34, 1049 (1978) ausführlich beschrieben.

Es wurde gefunden, daß die Verbindungen der allgemeinen Formen III, IV oder V oder deren Gemische mit 1 - 3 Äquivalenten Selendioxyd in die Verbindungen der allgemeinen Formel I übergeführt werden können.

Die Umsetzung wird in einem Gemisch von Wasser und einem organischen Lösungsmittel durchgeführt. Als organische Lösungsmittel können z.B. verschiedene Äther - wie Dioxan, Tetrahydrofuran oder Dimethoxyäthaneingesetzt werden. Die Reaktionstemperatur liegt im Bereich von 20-100°C, vorzugsweise von 25 - 100°C. Die Umsetzung ist nach 1 - 8 Stunden beendet.

Aus dem erhaltenen Reaktionsgemisch können die Verbindungen der allgemeinen Formel I durch Säulenchromatographie in einfacher Weise isoliert werden.

Unter der in der Beschreibung verwendeten Bezeichnung "pharmazeutisch verträgliches Kation" sind ein-, zwei- oder dreiwertige Kationen zu verstehen, die im lebenden Organismus in den Dosen, in denen die Verbindungen der allgemeinen Formel I angewandt werden, keine unerwünschten Nebenwirkungen hervorrufen. Einige Beispiele hierfür sind: die Kationen der Alkalimetalle (z.B. Natrium, Kalium, Lithium), der Erdalkalimetalle (z.B. Calcium, Magnesium), Aluminium, das Ammoniumion und die aus verschiedenen ein- oder mehrwertigen organischen Aminen ableitbaren ein- oder mehrwertigen Ammoniumionen (z.B. das Tris-(hydroxymetnyl)-ammonium-ion).

Unter "geradkettigem oder verzweigtem $C_{1-5}$-Alkyl" sind z.B. Methyl, Äthyl, n- oder Isopropyl, n-Butyl, Isobutyl, sec. Butyl, t-Butyl oder verschiedene Amylgruppen zu verstehen. $C_{1-8}$-Alkyl umfaßt außer den oben erwähnten $C_{1-5}$-Alkylgruppen auch die verschiedenen Hexyl-, Heptyl- und Octylgruppen.

Unter "$C_{1-5}$-Alkanoyl" sind aus $C_{1-5}$-Alkanen herleitbare Alkancarbonsäureradikale (z.B. Formyl, Acetyl, Propionyl und die verschiedenen Butyryl- und Valerianylgruppen) und unter "Aroyl" unsubstituiertes oder substituiertes Benzoyl und Naphthoyl zu verstehen. Diese Benzoyl- und Naphthoyl-Gruppen können z.B. als Substituenten ein oder mehrere Halogenatome enthalten.

Unter "gegebenenfalls monosubstituiertem Aryloxymethyl" ist im Ring gegebenenfalls durch ein oder mehrere Halogenatome substituiertes Phenoxymethyl und Naphthyloxymethyl zu verstehen.

Weitere Einzelheiten des Verfahrens der Erfindung sind den nachstehenden Beispielen zu entnehmen, ohne den Schutzumfang auf diese Beispiele einzuschränken.

**Beispiel 1**

Oxidation von 11,15-Diacetyl-6-methoxy-$PGI_1$-methylester mit Selendioxyd

2,388 g (4,95 mM) 11,15-Diacetyl-6-methoxy-$PGI_1$-methylester löst man in 10 ml eines Dioxan-Wasser-Gemisches (9: 1) und gibt bei 80°C unter Rühren in einer Portion 0,66 g (5,94 mM) Selendioxyd zu. Die Lösung nimmt nach 5 Minuten wegen des ausgeschiedenen Selens eine rötlichbraune Farbe an. Nach einer gewissen Zeit bildet das ausgeschiedene Selen ein schwarzes Granulat.

Der Ablauf der Reaktion kann durch Dünnschichtchromatographie verfolgt werden. Die $R_f$-Werte der Ausgangsverbindung und des gewünschten Produkts betragen 0,46 bzw. 0,29 (auf Silicagel; Laufmittel: 50 % Äthylacetat enthaltendes Hexan). Die Oxydation ist innerhalb von 1,5 - 2 Stunden beendet. Das Reaktionsgemisch wird auf Raumtemperatur gekühlt. Nach Zugabe von 1,5 ml Triäthylamin wird mit 150 ml Äthylacetat verdünnt, in einem Scheidetrichter zweimal mit je 10 ml Wasser und mit 15 ml einer gesättigten Natriumchloridlösung gewaschen und über Magnesiumsulfat getrocknet. Nach einer anderen Aufarbeitungsmethode wird das Reaktionsgemisch mit Aktivkohle geklärt, filtriert, abgekühlt, mit 150 ml Äthylacetat verdünnt, in einem 1 Liter-Scheidetrichter nacheinander zweimal mit je 10 ml Wasser, 3 ml einer gesättigten Natriumbicarbonatlösung, 20 ml Wasser und 15 ml einer gesättigten Natriumchloridlösung gewaschen und über Magnesiumsulfat getrocknet.

Nach Abdestillieren des Lösungsmittels wird der Rückstand auf einer Silicagelsäule (350 g) chromatographiert und mit einem n-Hexan/Äthylacetat Gemisch (1: 1) eluiert. Es werden - in Form einer strohgelben viskosen Flüssigkeit - 1,52 g 11, 15-Diacetyl-6-hydroxy-7-oxo-$PGI_1$-methylester erhalten, Ausbeute: 63 %.

$R_f$ = 0,29 (Silicagelschicht, Hexan-Äthylacetat 1: 1),

IR (Film): $\gamma_{max}$ 3250 - 3100 (breites Band, OH),

1730 (C=0D) $cm^{-1}$.

**Beispiel 2**

Oxydation eines Gemisches von 11,15-Diacetyl-$PGI_2$-methylester und 11, 15-Diacetyl- $\Delta^6$ -$PGI_1$-methylester mit Selendioxyd

0,215 g (0,477 mM) eines Gemisches von 11, 15-Diacetyl-$PGI_2$-methylester und 11, 15-Diacetyl- $\Delta^6$-$PGI_1$-methylester löst man in 2 ml eines Tetrahydrofuran-Wasser-Gemisches (9: 1), gibt der Lösung unter Rühren auf einmal 0,106 g (0,954 mM) Selendioxyd zu und erhitzt das Gemisch 1,5-2 Stunden unter Rückfluß.

Der Reaktionsablauf kann durch Dünnschichtchromatographie verfolgt werden. Die $R_f$-Werte der Ausgangsverbindungen und des gewünschten Produkts betragen 0,54 bzw. 0,29 (Laufmittel: 1: 1 n-Hexan/Äthylacetat-Gemisch). Nach Beendigung der Reaktion wird das Gemisch auf Raumtemperatur abgekühlt, nach Zugabe von 0,5 ml Triäthylamin mit 100 ml Äthylacetat verdünnt, zweimal mit je 10 ml Wasser und 15 ml einer gesättigten Natriumchloridlösung gewaschen und über Magnesiumsulfat getrocknet.

Das Lösungsmittel wird abdestilliert und der Rückstand auf einer 30 g Silicagel enthaltenden Säule chromatographiert und mit 50 % Äthylacetat enthallendem Hexan eluiert. Es werden 0,13 g des 11, 15-Diacetyl-6-hy-droxy-7-oxo-$PGI_1$-methylesters erhalten, Ausbeute 59 %.

$R_f$ = 0,28 (n-Hexan/Äthylacetat 1: 1).

IR (Film: $\gamma_{max}$ 3250 - 3100 (breites Band, OH)

1730 (C=0) $cm^{-1}$.

**Beispiel 3**

Oxydation des 11, 15-Diacetyl-6-hydroxy-$PGI_1$-methylesters mit Selendioxyd

0,5 g (1 mM) 11, 15-Diacetyl-6-hydroxy-$PGI_1$-methylester werden mit der 1, 2 moläquivalenten Menge von Selendioxyd in der in Beispiel 2 angegebenen Weise oxydiert. Es werden 0,29 g 11, 15-Diacetyl-6-hydroxy-7-oxo-$PGI_1$-methylester erhalten. Ausbeute: 58 %. $R_f$ = 0,20.

**Beispiel 4**

Herstellung des 11, 15-Diacetyl-6-methoxy-7-oxo-$PGI_1$-methylesters

0,074 g (0,15 mM) 11, 15-Diacetyl-6-hydroxy-7-oxo-$PGI_1$-methylester löst man in 3 ml Methanol, gibt unter Rühren bei Raumtemperatur einen Tropfen Bortrifluoridätherat und nach 15 Minuten Rühren 1 ml Triäthylamin zu, worauf das Methanol in einem Rotationsverdampfer unter vermindertem Druck entfernt wird. Das als Rückstand erhaltene Öl wird auf einer 20 g Silicagel enthaltenden Säule chromatographiert und mit 30 % Äthylacetat und 0,5 % Triäthylamin enthaltendem n-Hexan eluiert.

Es werden 0,056 g 11, 15-Diacetyl-6-methoxy-7-oxo-$PGI_1$-methylester erhalten. Ausbeute: 73 %. $R_f$ = 0,34 (Laufmittel: n-Hexan/Äthylacetat 2: 1, zweimal verwendet).

**Patentansprüche** für die Vertragsstaaten BE, IT

1. Verfahren zur Herstellung von 6-substituierten 7-Oxo-PGI-Derivaten der allgemeinen Formel

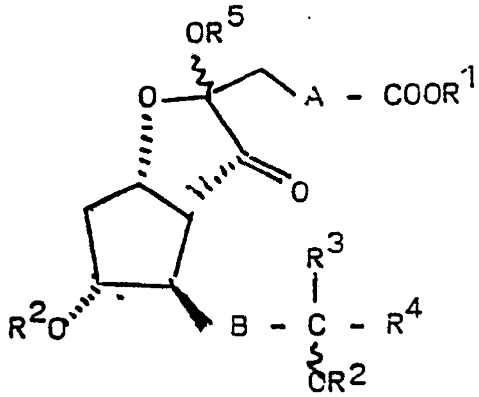

(I) ,

worin
A geradkettiges oder verzweigtes $C_{1-5}$-Alkylen,
B Äthylen, Z- oder E-Vinylen oder Äthinylen,
$R^1$ Wasserstoff, $C_{1-5}$-Alkyl oder ein pharmazeutisch verträgliches Kation,
$R^2$ Wasserstoff, $C_{1-5}$-Alkanoyl oder Aroyl,
$R^3$ Wasserstoff oder Methyl,
$R^4$ geradkettiges oder verzweigtes $C_{1-8}$-Alkyl oder gegebenenfalls monosubstituiertes Aryloxymethyl und
$R^5$ Wasserstoff oder $C_{1-5}$-Alkyl bedeuten, dadurch gekennzeichnet, daß man eine Verbindung der allgemeinen Formel

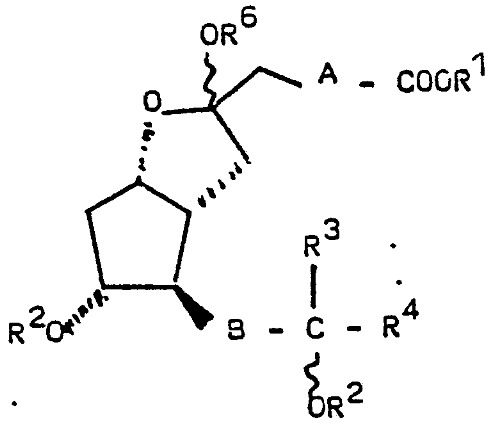

(III),

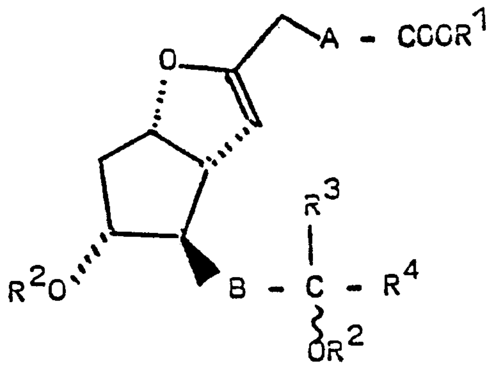

(IV)

oder

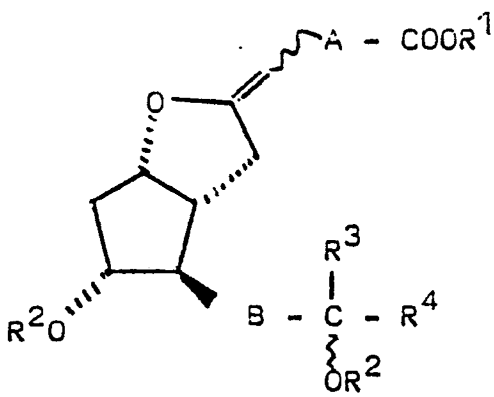

(V),

worin

A, B, $R^1$, $R^2$, $R^3$ und $R^4$ die oben angegebene Bedeutung haben und $R^6$ Wasserstoff oder $C_{1-5}$-Alkyl oder -Alkanoyl bedeutet, oder ein Gemisch dieser Verbindungen mit einem milden elektrophilen Oxydationsmittel oxydiert und die erhaltene Verbindung der allgemeinen Formel I, in der $R^5$ Wasserstoff ist und A, B, $R^1$, $R^2$, $R^3$ und $R^4$ die oben angegebene Bedeutung haben, entweder als solche isoliert oder in an sich bekannter Weise mit einem $C_{1-5}$-Alkanol in Gegenwart von Bortrifluorätherat in die entsprechende Verbindung der allgemeinen Formel 1, in der $R^5$ $C_{1-5}$-Alkyl bedeutet, überführt.

2. Verfahren nach Anspruch 1, dadurch gekennzeichnet, daß man als Oxydationsmittel 1 - 3 Äquivalente Selendioxyd verwendet.

3. Verfahren nach Anspruch 1 oder 2, dadurch gekennzeichnet, daß man die Umsetzung in einem Gemisch von Wasser und einem Äther durchführt.

4. Verfahren nach Anspruch 3, dadurch gekennzeichnet, daß man als Lösungsmittel ein Gemisch von Wasser und Dioxan, Wasser und Tetrahydrofuran oder Wasser und Dimethoxyäthan verwendet.

5. Verfahren nach Anspruch 1, 2, 3 oder 4, dadurch gekennzeichnet, daß man die Umsetzung bei einer Temperatur im Bereich von 20 bis 100° C durchführt.

**Patentansprüche** für die Vertragsstaaten DE, FR, GB, LU, NL

1. Verfahren zur Herstellung von 6-substituierten 7-Oxo-PGI-Derivaten der allgemeinen Formel

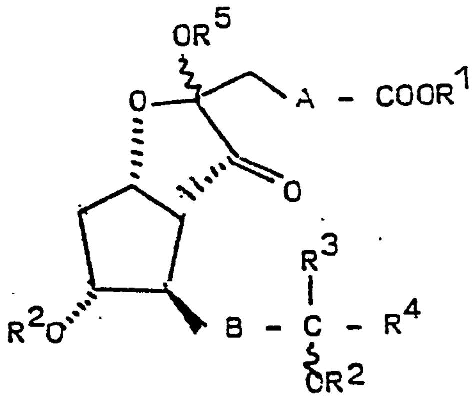

(I) ,

worin
A geradkettiges oder verzweigtes $C_{1-5}$-Alkylen,
B Äthylen, Z- oder E-Vinylen oder Äthinylen,
$R^1$ Wasserstoff, $C_{1-5}$-Alkyl oder ein pharmazeutisch verträgliches Kation,
$R^2$ Wasserstoff, $C_{1-5}$-Alkanoyl oder Aroyl,
$R^3$ Wasserstoff oder Methyl,
$R^4$ geradkettiges oder verzweigtes $C_{1-8}$-Alkyl oder gegebenenfalls monosubstituiertes Aryloxymethyl und
$R^5$ Wasserstoff oder $C_{1-5}$-Alkyl bedeuten, dadurch gekennzeichnet, daß man eine Verbindung der allgemeinen Formel

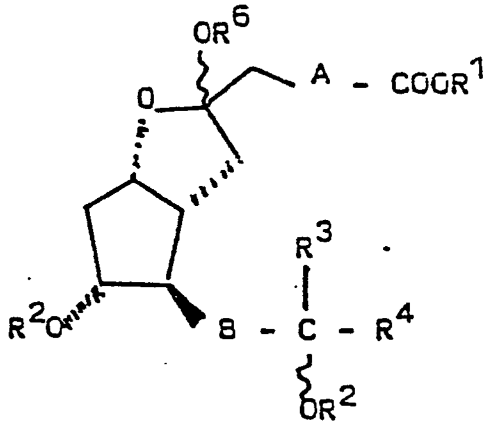

(III),

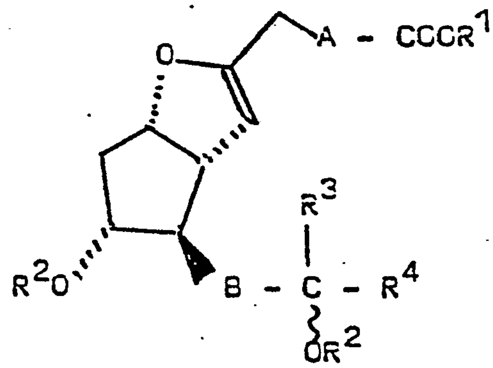

(IV)

oder

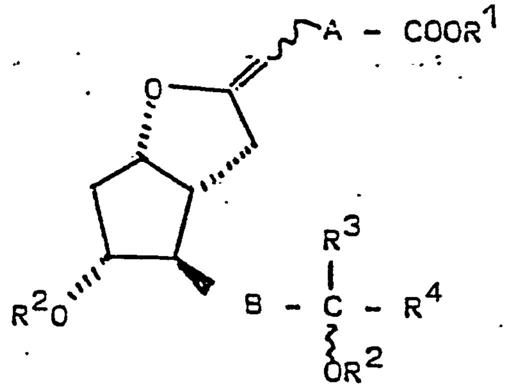

(V);

worin

A, B, $R^1$, $R^2$, $R^3$ und $R^4$ die oben angegebene Bedeutung haben und $R^6$ Wasserstoff oder $C^{1-5}$-Alkyl oder -Alkanoyl bedeutet, oder ein Gemisch dieser Verbindungen mit einem milden elektrophilen Oxydationsmittel in einem Gemisch von Wasser und einem organischen Lösungsmittel oxydiert und die erhaltene Verbindung der allgemeinen Formel I, in der $R^5$ Wasserstoff ist und A, B, $R^1$, $R^2$, $R^3$ und $R^4$ die oben angegebene Bedeutung haben, entweder als solche isoliert oder in an sich bekannter Weise mit einem $C^{1-5}$-Alkanol in Gegenwart von Bortrifluorätherat in die entsprechende Verbindung der allgemeinen Formel I, in der $R^5$ $C_{1-5}$-Alkyl bedeutet, überführt.

2. Verfahren nach Anspruch 1, dadurch gekennzeichnet, daß man als Oxydationsmittel 1 - 3 Äquivalente Selendioxyd verwendet.

3. Verfahren nach Anspruch 1 oder 2, dadurch gekennzeichnet, daß man als organisches Lösungsmittel Äther, wie Dioxan, Tetrahydrofuran oder Dimethoxyäthan, verwendet.

4. Verfahren nach Anspruch 1, 2 oder 3, dadurch gekennzeichnet, daß man die Umsetzung bei einer Temperatur im Bereich von 20 bis 100°C durchführt.

**Claims** for the contracting States BE, IT

1. Process for the preparation of 6-substituted 7-oxo-PGI derivatives of general formula

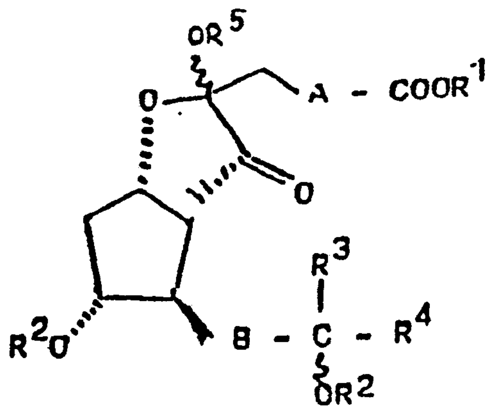

(I)

in which
A stands for straight or branched-chain $C_{1-5}$ alkylene
B for ethylene, Z or E-vinylene or ethynylene
$R^1$ for hydrogen, $C_{1-5}$ alkyl or a pharmaceutically acceptable cation
$R^2$ for hydrogen, $C_{1-5}$ alkanoyl or aroyl
$R^3$ for hydrogen or methyl
$R^4$ for straight or branched-chain $C_{1-8}$ alkyl or optionally monosubstituted aryloxymethyl and
$R^5$ for hydrogen or $C_{1-5}$ alkyl,
characterized in that, a compound of general formula

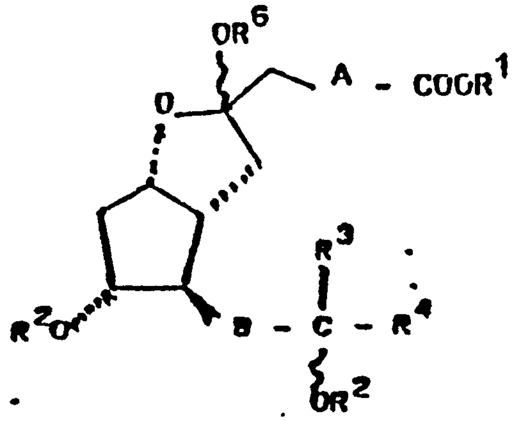

(III)

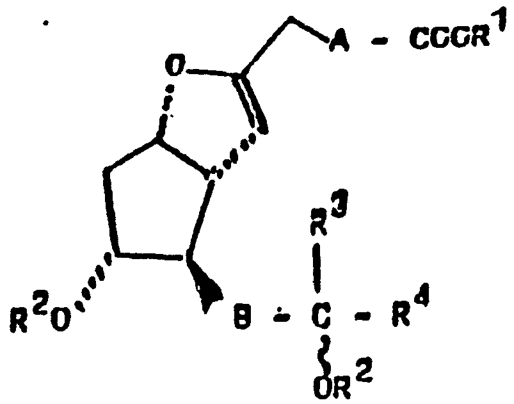

(IV)

or

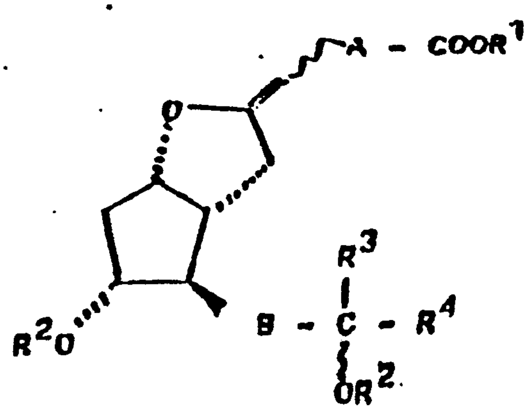

(V)

in which

A, B, $R^1$, $R^2$, $R^3$, and $R^4$ have the above meanings and $R^6$ stands for hydrogen or $C_{1-5}$ alkyl or alkanoyl, or a mixture of these compounds is oxidized with a mild electrophilic oxidizing agent and the compound of general formula I obtained, in which $R^5$ is hydrogen and A, B, $R^1$, $R^2$, $R^3$, and $R^4$ have the above meanings, is either isolated as such, or is converted in per se known manner into the corresponding compound of the general formula I, in which $R^5$ is $C_{1-5}$ alkyl, with a $C_{1-5}$ alkanol in the presence of boron trifluoride etherate.

2. Process according to claim 1, characterized in that, 1 - 3 equivalents of selenium dioxide is used as the oxidizing agent.

3. Process according to claims 1 or 2, characterized in that, the reaction is performed in a mixture of water

and an ether.

4. Process according to claim 3, characterized in that, a mixture of water and dioxan, water and tetrahydrofuran or water and dimethoxyethane is used as the solvent.

5. Process according to claims 1, 2, 3 or 4, chsracterized in that, the reaction is performed at a temperature in the range 20 to 100° C.

**Claims** for the contracting States DE, FR, GB, LU, NL

Process for the preparation of 6-substituted 7-oxo-PGI derivatives of general formula

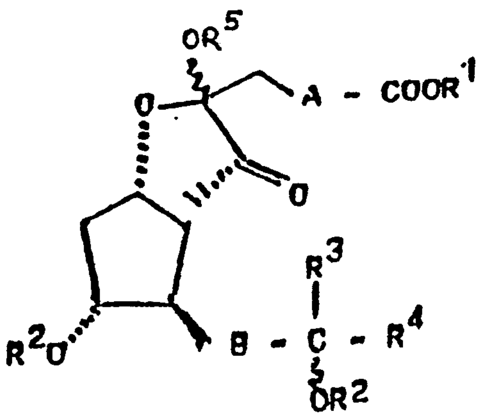

(I)

in which
A stands for straight or branched-chain $C_{1-5}$ alkylene
B for ethylene Z or E-vinylene or ethynylene
$R^1$ for hydrogen, $C_{1-5}$ alkyl or a pharmaceutically acceptable kation
$R^2$ for hydrogen, $C_{1-5}$ alkanoyl or aroyl
$R^3$ for hydrogen or methyl
$R^4$ for straight or branched-chain $C_{1-8}$ alkyl or optionally monosubstituted aryloxymethyl and
$R^5$ for hydrogen or $C_{1-5}$ alkyl,
characterized in that, a compound of general formula

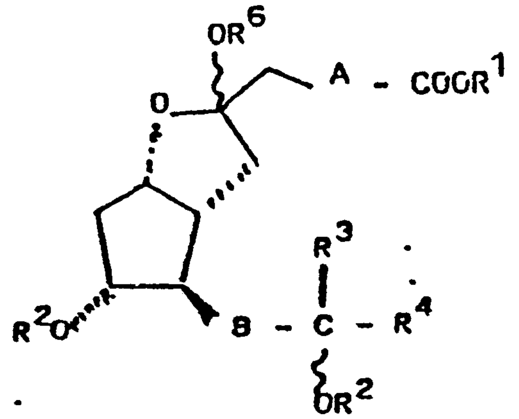

(III)

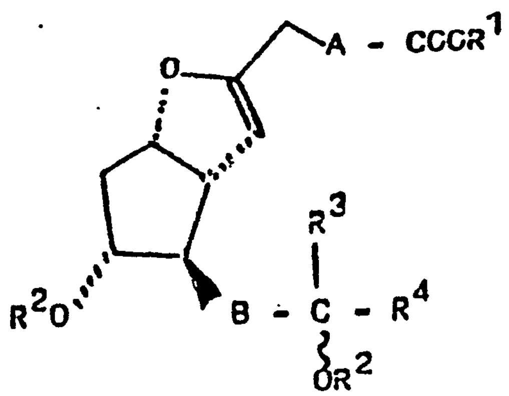

(IV)

or

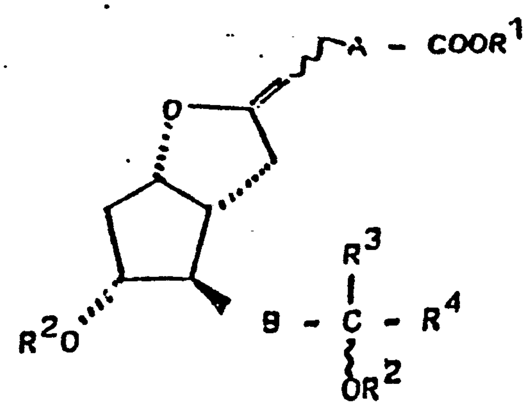

(V)

in which

A, B, $R^1$, $R^2$, $R^3$, and $R^4$ have the above meanings and $R^6$ stands for hydrogen or $C_{1-5}$ alkyl or alkanoyl or a mixture of these compounds is oxidized with a mild electrophilic oxidizing agent in a mixture of water and an organic solvent and the compound obtained of general formula I, in which $R^5$ is hydrogen and A, B, $R^1$, $R^2$, $R^3$, and $R^4$ have the above meanings, is either isolated as such or is converted in per se known manner into the corresponding compound of general formula I, in which $R^5$ is $C_{1-5}$ alkyl, with a $C_{1-5}$ alkanol in the presence of boron trifluoride etherate.

2. Process according to claim 1, characterized in that, 1-3 equivalents of selenium dioxide is used as the oxidizing agent.

3. Process according to claims 1 or 2, characterized in that, ether, dioxan, tetrahydrofuran or dimethoxyethane is used as the organic solvent.

4. Process according to claims 1, 2 or 3, characterized in that, the reaction is performed at a temperature in the range 20 to 100° C.

**Revendications** pour les Etats contractant BE, IT

1. Procédé de préparation de dérivés de 7-oxo-PGI 6-substitués de formule générale

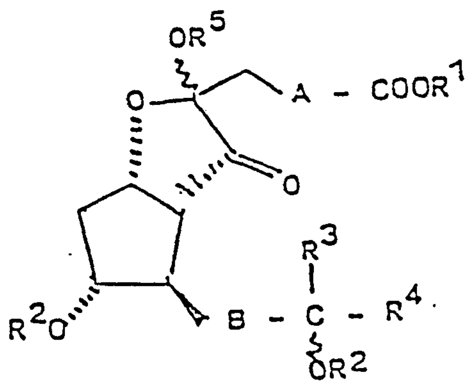

(I) ,

où

A représente un alcoylène en C1 à C5 a chaîne droite ou ramifiée,

B représente un éthylène, un 2- ou E-vinylène ou un éthynylène,

R1 représente un hydrogène, un alcoyle en C1 à C5 ou un cation pharmaceutiquement acceptable.

R2 représente un hydrogène, un alcanoyle en C1 à C5 ou un aroyle,

R3 représente un hydrogène ou un méthyle,

R4 représente un alcoyle en C1 à C8 à chaîne droite ou ramifiée ou un aryloxyméthyle éventuellement monosubstitué et

R5 représente un hydrogène ou un alcoyle en C1 à C5, caractérisé en ce qu'on oxyde un composé de formule générale

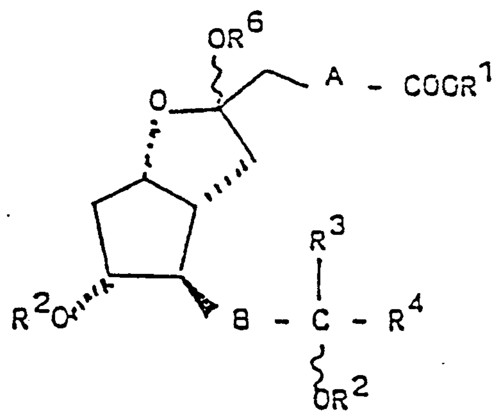

(III),

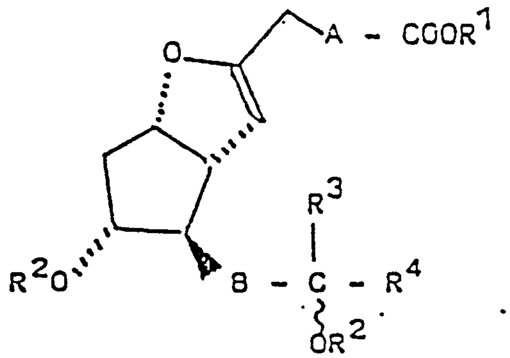

(IV)

ou

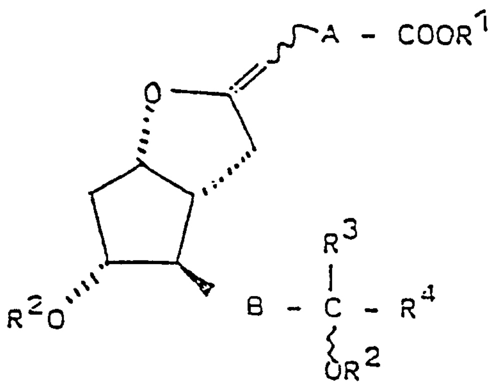

(V),

où

A, B, R1. R2, R3 et R4 ont la signification donnée ci-dessus et R6 représente un hydrogène ou un alcoyle ou alcanoyle en C1 à C5, ou un mélange de ces composés avec un oxydant életrophile doux, et en ce qu'on isole le composé obtenu de formule générale I, où R5 est un hydrogène et A, B, R1, R2, R3 et R4 ont la signification donnée ci-dessus, tel quel, ou en ce qu'on le transforme de façon connue avec un alcanol en C1 à C5 en présence d'éthérate de trifluorure de bore, en le composé correspondant de formule générale I où R5 représente un alcoyle en C1 à C5.

2. Procédé selon la revendication 1, caractérisé en ce qu'on utilise comme oxydant de 1 à 3 équivalents de dioxyde de sélénium.

3. Procédé selon les revendications 1 et 2, caractérisé en ce qu'on conduit la réaction dans un mélange d'eau/et d'un éther.

4. Procédé selon la revendication 3, caractérisé en ce qu'on utilise comme solvant un mélange d'eau et de dioxanne, d'eau et de tétrahydrofuranne ou d'eau et de diméthoxyéthane.

5. Procédé selon les revendications 1, 2, 3 et 4, caractérisé en ce qu'on conduit la réaction à une température située dans un intervalle allant de 20 à 100° C.

**Revendications** pour les Etats contractant DE, FR, GB, LU, NL

1. Procédé de préparation de dérivés de 7-oxo-PGI 6-substitués de formule générale

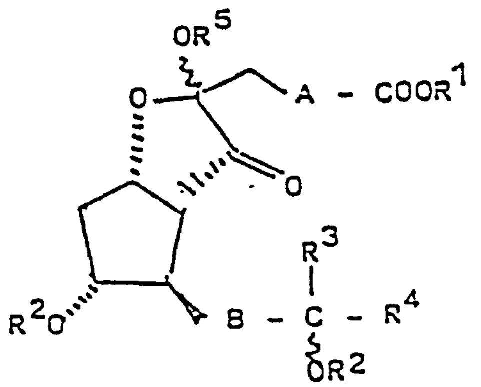

(I) ,

où
A représente un alcoylène en C1 à C5 à chaîne droite ou ramifiée,
B représente un éthylène, un Z- ou E-vinylène ou un éthynylène,
R1 représente un hydrogène, un alcoyle en C1 à C5 ou un cation pharmaceutiquement acceptable,
R2 représente un hydrogène, un alcanoyle en C1 à C5 ou un aroyle,
R3 représente un hydrogène ou un méthyle,
R4 représente un alcoyle en C1 à C8 à chaîne droite ou ramifiée ou un aryloxyméthyle éventuellement monosubstitué et
R5 représente un hydrogène ou un alcoyle en C1 à C5
caractérisé en ce qu'on oxyde un composé de formule générale

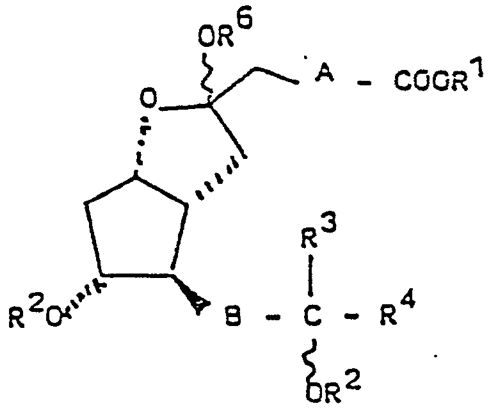

(III).

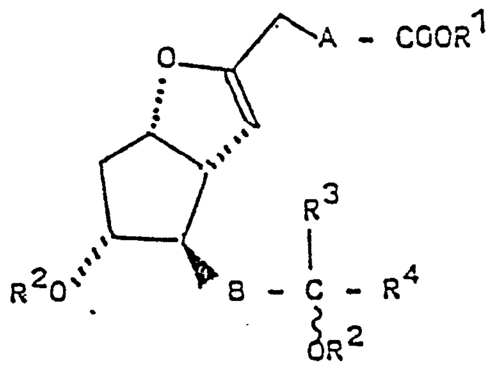

(IV)

ou

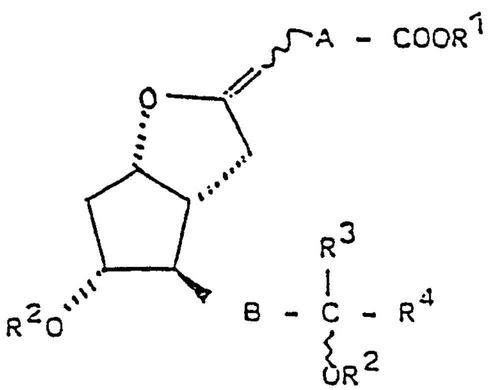

(V),

où

A, B, R1, R2, R3 et R4 ont la signification donnée ci-dessus et R6 représente un hydrogène ou un alcoyl ou alcanoyle en C1 à C5, ou un mélange de ces composés avec un oxydant électrophile doux, dans un mélange d'eau et d'un solvant organique, et en ce que ou bien on isole tels quels les composés obtenus de formule générale I où R5 est un hydrogène et A, B, R1, R2, R3 et R4 ont la signification donnée ci-dessus, ou on les transforme de façon connue avec un alcanol en C1 à C5 en présence d'éthérate de trifluorure de bore en le composé correspondant de formule generale I où R5 représente un alcoyle en C1 à C5.

2. Procédé selon la revendication 1, caractérisé en ce qu'on utilise comme oxydant de 1 à 3 équivalents de dioxyde de sélénium.

3. Procédé selon les revendications 1 et 2, caractérisé en ce qu'on utilise comme solvant organique des éthers comme le dioxanne, le tétrahydrofuranne ou le diméthoxyéthane.

4. Procédé selon les revendications 1, 2 et 3, caractérisé en ce qu'on conduit la réaction à une température située dans un intervalle allant de 20 à 100° C.